# EUROPÄISCHE PATENTSCHRIFT

(11) **EP 3 147 289 B1**
(45) Veröffentlichungstag und Bekanntmachung des Hinweises auf die Patenterteilung: **22.11.2017**
(21) Anmeldenummer: 15197686.7
(22) Anmeldetag: 03.12.2015
(51) Int. Cl.: C07F 9/6574, C07C 45/50

(54) **MONOPHOSPHITE, DIE EIN TEILHYDRIERTES BINOL-DERIVAT AUFWEISEN**
MONOPHOSPHITES COMPRISING A PARTIALLY HYDRATED BINOL DERIVATIVE
MONOPHOSPHITES PRESENTANT UN DERIVE DU BINOL PARTIELLEMENT HYDROGÈNE

(30) Priorität: 22.09.2015 EP 15186166
(43) Veröffentlichungstag der Anmeldung: 29.03.2017
(73) Patentinhaber: Evonik Degussa GmbH, 45128 Essen (DE)
(72) Erfinder: BÖRNER, Armin, 18059 Rostock (DE); DYBALLA, Katrin Marie, 45657 Recklinghausen (DE); FRANKE, Robert, 45772 Marl (DE); FRIDAG, Dirk, 45721 Haltern (DE); GEILEN, Frank, 45721 Haltern (DE); HESS, Dieter, 45770 Marl (DE); HÄGER, Harald, 59348 Lüdinghausen (DE); SELENT, Detlef, 18059 Rostock (DE)

(56) Entgegenhaltungen:
- WO-A2-2004/076464
- CN-A- 104 744 514
- US-A1- 2009 182 164
- GAVRILOV K N ET AL: "Facile one-pot synthesis of BINOL- and H8-BINOL-based aryl phosphites and their use in palladium catalysed asymmetric allylation", TETRAHEDRON, ELSEVIER SCIENCE PUBLISHERS, AMSTERDAM, NL, Bd. 61, Nr. 44, 31. Oktober 2005 (2005-10-31), Seiten 10514-10520, XP027861502, ISSN: 0040-4020 [gefunden am 2005-10-31]
- ROSALBA BELLINI ET AL: "Coordination Studies on Supramolecular Chiral Ligands and Application in Asymmetric Hydroformylation", CHEMISTRY - A EUROPEAN JOURNAL., Bd. 18, Nr. 23, 4. Juni 2012 (2012-06-04), Seiten 7091-7099, XP055228886, WEINHEIM, DE ISSN: 0947-6539, DOI: 10.1002/chem.201200225
- DEYUN QIAN ET AL: "Gold(I)-Catalyzed Highly Diastereo- and Enantioselective Alkyne Oxidation/Cyclopropanation of 1,6-Enynes", ANGEWANDTE CHEMIE INTERNATIONAL EDITION, Bd. 53, Nr. 50, 8. Dezember 2014 (2014-12-08), Seiten 13751-13755, XP055228894, DE ISSN: 1433-7851, DOI: 10.1002/anie.201407717
- HUGO TRICAS ET AL: "Bulky monophosphite ligands for ethene hydroformylation", JOURNAL OF CATALYSIS, Bd. 298, 1. Februar 2013 (2013-02-01), Seiten 198-205, XP055185769, ISSN: 0021-9517, DOI: 10.1016/j.jcat.2012.11.031
- ROBERT FRANKE ET AL: "Applied Hydroformylation", CHEMICAL REVIEWS, Bd. 112, Nr. 11, 14. November 2012 (2012-11-14), Seiten 5675-5732, XP055091930, ISSN: 0009-2665, DOI: 10.1021/cr3001803

## Beschreibung

Die Erfindung betrifft Monophosphite, die ein teilhydriertes Derivat von 1,1'-Bi-2-naphthol (BINOL) aufweisen, sowie deren Verwendung als Liganden für Hydroformylierungskatalysatoren.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigen Phosphor PIII. Eine gute Übersicht über den Stand der Hydroformylierung von Olefinen findet sich in B. Cornils, W. A. Herrmann, "Applied Homogeneous Catalysis with Organometallic Compounds", Vol. 1 & 2, VCH, Weinheim, New York, 1996 bzw. R. Franke, D. Selent, A. Börner, "Applied Hydroformylation", Chem. Rev., 2012, DOI:10.1021/cr3001803.

Jede katalytisch aktive Zusammensetzung hat ihre spezifischen Vorzüge. Je nach Einsatzstoff und Zielprodukt kommen daher unterschiedliche katalytisch aktive Zusammensetzungen zum Einsatz.

Die Hydroformylierung führt, außer im Fall von Ethylen als Edukt, zu einer Mischung isomerer Produkte, nämlich n-Aldehyden (lineare Aldehyde) und iso-Aldehyden (verzweigte Aldehyde). Neben der Reaktionsgeschwindigkeit ist somit auch die Selektivität bei der Bildung von n- bzw. iso-Produkten ein wichtiger Parameter der Hydroformylierungsreaktion.

Die einfache Verfügbarkeit und damit verbunden die gute Möglichkeit eines großtechnischen Einsatzes ist ein wichtiges Kriterium, da der Herstellungsaufwand und damit verbunden die anfallenden Kosten nur so hoch sein dürfen, dass die Rentabilität des Gesamtprozesses weiterhin gewährleistet ist.

Rhodium-Monophosphit-Komplexe in katalytisch aktiven Zusammensetzungen sind geeignet für die Hydroformylierung von verzweigten Olefinen mit innenständigen Doppelbindungen.

Seit den 1970er Jahren ist die Verwendung von so genannten "bulky phosphites" in der Hydroformylierungbeschrieben (siehe u.a. van Leeuwen et. al, Journal of Catalysis, 2013, 298, 198-205). Diese zeichnen sich durch eine gute Aktivität aus, jedoch ist die n/i-Selektivität für endständig oxierte Verbindungen gering und verbesserungswürdig.

Aus EP 0 155 508 A1 ist die Verwendung von bisarylensubstituierten Monophosphiten bei der rhodiumkatalysierten Hydroformylierung von sterisch gehinderten Olefinen, z. B. Isobuten, bekannt. Hierbei werden jedoch z.T. sehr hohe Rhodiumkonzentrationen verwendet (u.a. 250 ppm), was in Anbetracht des derzeitigen Rhodiumpreises für ein großtechnisches Verfahren inakzeptabel ist und verbessert werden muss.

Für Hydroformylierungsreaktionen ist derzeit Tris(2,4-di-tert-butylphenyl)phosphit (TDTBPP) einer der leistungsstärksten und kommerziell verfügbaren Monophosphitliganden, welcher unter dem Handelsnamen Alkanox 240 erhältlich ist (siehe auch R. Franke, D. Selent, A. Börner, "Applied Hydroformylation", Chem. Rev., 2012, 112, S.5681 Kapitel 3.4.2).

CHEMISTRY - A EUROPEAN JOURNAL., Bd. 18, Nr. 23, 2012, Seiten 7091-7099 offenbart Verbindungen (S)-1c-(S)-1g und ihre Verwendung in der Hydroformylierung.

JOURNAL OF CATALYSIS, Bd. 298, 2013, Seiten 198-205 lehrt die Verwendung sterisch anspruchsvoller Liganden in der Hydroformylierung. Der vorliegenden Erfindung lag vor diesem Hintergrund die Aufgabe zugrunde, neue Liganden für die Hydroformylierung bereitzustellen, welche eine hohe Ausbeute bei gleichzeitig hoher n-Selektivität gewährleisten. Offenbart wird eine Verbindung gemäß Formel (I): 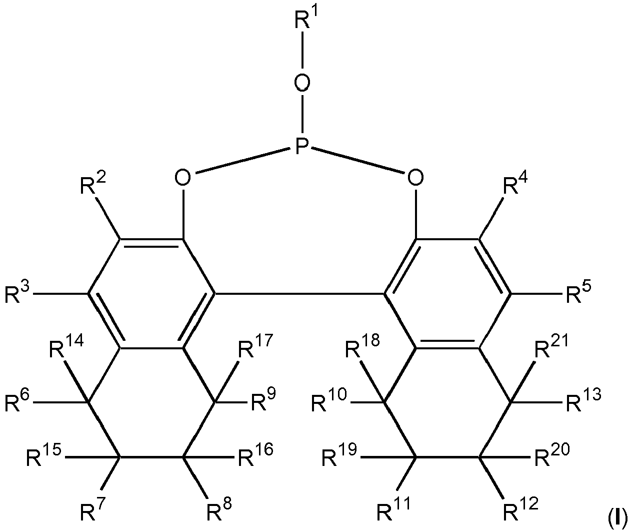
wobei R¹ Anthracenyl oder Diphenylphenyl darstellt;
R² bis R²¹ unabhängig voneinander ausgewählt sein können aus -H, -(C₆-C₂₀)-Aryl, -(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₃-C₁₂)-Cycloalkyl, -N-[(C₁-C₁₂)-Alkyl]₂, -COOH, -OH, -SO₃H, -NH₂, und Halogen.

Die offenbarten Phosphite gemäß Formel (I), welche mit einem teilhydriertem Derivat von 1,1 '-Bi-2-naphthol substituiert sind, eignen sich in besonderer Weise als Liganden für Hydroformylierungskatalysatoren. Unter Verwendung dieser Liganden lassen sich hohe Ausbeuten sowie eine hohe Selektivität bei der Herstellung von n-Aldehyden aus endständigen Olefinen erzielen. Insbesondere hat sich dies bei der Hydroformylierung von n-Octen gezeigt. Diese Vorteile lassen sich außerdem bei einem relativ geringen Verhältnis von Ligand zu katalytisch aktivem Edelmetall, z.B. Rh, erzielen.

Bei die Binaphthol bzw. Octahydrodinaphtholgruppe kann eine *S*- oder *R*-Konfiguration aufweisen und auch als Racemat vorliegen.

Die beiden Naphtholgruppen bzw. teilhydrierten Naphtholgruppen mit den Substituenten R² bis R²¹ können symmetrisch oder asymmetrisch substituiert sein. In einer bevorzugten Ausführungsform sind sie symmetrisch substituiert.

Im Rahmen der Offenbarung umfasst der Ausdruck -(C₁-C₁₂)-Alkyl geradkettige und verzweigte Alkylgruppen mit 1 bis 12 Kohlenstoffatomen. Vorzugsweise handelt es sich dabei um geradkettige oder verzweigte -(C₁-C₈)-Alkyl- und ganz bevorzugt um geradkettige oder verzweigte -(C₁-C₆)-Alkylgruppen. Beispiele für -(C₁-C₁₂)-Alkylgruppen sind insbesondere Methyl, Ethyl, Propyl, Isopropyl, n-Butyl, iso-Butyl, sec.-Butyl, tert.-Butyl, n-Pentyl, 2-Pentyl, 2-Methylbutyl-, 3-Methylbutyl-, 1,2-Dimethylpropyl-, 1,1-Dimethylpropyl, 2,2-Dimethylpropyl-, 1-Ethylpropyl-, n-Hexyl-, 2-Hexyl-, 2-Methylpentyl-, 3-Methylpentyl-, 4-Methylpentyl-, 1,1-Dimethylbutyl-, 1,2-Diemthylbutyl-, 2,2-Dimethylbutyl-, 1,3-Dimethylbutyl-, 2,3-Dimethylbutyl-, 3,3-Dimethylbutyl-, 1,1,2-Trimethylpropyl-, 1,2,2-Trimethylpropyl-, 1-Ethylbutyl-, 1-Ethyl-2-methylpropyl-, n-Heptyl-, 2-Heptyl-, 3-Heptyl-, 2-Ethylpentyl-, 1-Propylbutyl-, n-Octyl-, 2-Ethylhexyl-, 2-Propylheptyl-, Nonyl-, Decyl.

Die Erläuterungen zum Ausdruck -(C₁-C₁₂)-Alkyl gelten auch für die Alkylgruppen in -O-(C₁-C₁₂)-Alkyl, -S-(C₁-C₁₂)-Alkyl, -COO-(C₁-C₁₂)-Alkyl, -CONH-(C₁-C₁₂)-Alkyl, -CO-(C₁-C₁₂)-Alkyl, und -N-[(C₁-C₁₂)-Alkyl]₂.

Der Ausdruck -(C₃-C₁₂)-Cycloalkyl umfasst im Sinne der vorliegenden Erfindung zyklische Kohlenwasserstoffreste mit 3 bis 12, insbesondere 5 bis 12 Kohlenstoffatomen. Insbesondere handelt es sich dabei um mono-, bi-oder tricyclische Kohlenwasserstoffreste. Dazu zählen Cyclopropyl, Cyclobutyl, Cyclopentyl, Cyclohexyl, Cycloheptyl, Cyclooctyl, Cyclododecyl, Cyclopentadecyl, Norbonyl oder Adamantyl. Unter den Begriff -(C₃-C₁₂)-Cycloalkyl fallen auch alkylsubstituierte Cycloalkylgruppen mit insgesamt 3 bis 12 Kohlenstoffatomen, wie beispielsweise Menthyl.

Der Ausdruck -(C₃-C₁₂)-Heterocycloalkyl umfasst im Sinne der vorliegenden Erfindung nichtaromatische, gesättigte oder teilweise ungesättigte cycloaliphatische Gruppen mit 3 bis 12, insbesondere 5 bis 12, Kohlenstoffatomen. Die -(C₃-C₁₂)-Heterocycloalkylgruppen weisen vorzugsweise 3 bis 8, besonders bevorzugt 5 oder 6, Ringatome auf. In den Heterocycloalkylgruppen sind im Unterschied zu den Cycloalkylgruppen 1, 2, 3 oder 4 der Ringkohlenstoffatome durch Heteroatome oder heteroatomhaltige Gruppen ersetzt. Die Heteroatome oder die heteroatomhaltige Gruppen sind vorzugsweise ausgewählt unter -O-, -S-, -N-, -N(=O)-, -C(=O)- oder -S(=O)-. Beispiele für -(C₃-C₁₂)-Heterocycloalkylgruppen Tetrahydrothiophenyl, Tetrahydrofuryl, Tetrahydropyranyl und Dioxanyl.

Der Ausdruck -(C₆-C₂₀)-Aryl umfasst im Sinne der vorliegenden Erfindung mono- oder polycyclische aromatische Kohlenwasserstoffreste mit 6 bis 20 Kohlenstoffatomen. Geeignete Arylgruppen sind beispielsweise Phenyl, Naphthyl, Indenyl, Fluroenyl, Anthracenyl, Phenanthrenyl, Naphthacenyl, Chrysenyl, Pyrenyl, und Coronenyl.

Bei den genannten Halogen-Substituenten handelt es sich bevorzugt um Fluor oder Chlor oder Iod, bevorzugt Chlor oder Fluor.

Die Gruppe R¹ kann unsubstituiert oder mit einer oder mehrerer der erwähnten Gruppen substituiert sein.

In einer weiteren bevorzugten Ausführungsform stellt R¹ Anthracenyl oder Diphenylphenyl dar. Hierbei kommt als Diphenylphenyl vor allem 3,5-Diphenylphenyl in Betracht.

In einer Ausführungsform sind R² bis R²¹ unabhängig voneinander ausgewählt aus -H, -(C₁-C₁₂)-Alkyl, und -O-(C₁-C₁₂)-Alkyl, bevorzugt aus -H, -(C₁-C₆)-Alkyl, und -O-(C₁-C₆)-Alkyl.

In einer Ausführungsform stellen R² bis R²¹ jeweils -H dar.

In einer bevorzugten Ausführungsform stellt R¹ Anthracenyl oder Diphenylphenyl dar und stellen R² bis R²¹ jeweils -H dar. Die erfindungsgemäße Aufgabe wird gelöst durch die Verbindung ausgewählt aus einer der Strukturen gemäß den Formeln (**1**) und (**2**),

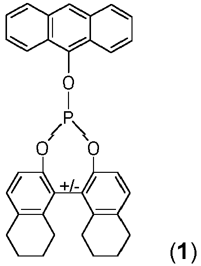

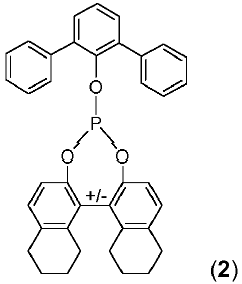

Neben den genannten Verbindungen betrifft die Erfindung auch einen Komplex, welcher die genannte Verbindung und ein Metallatom ausgewählt aus Rh, Ru, Co und Ir umfasst. In einer bevorzugten Ausführungsform ist das Metall Rh.

Die Herstellung derartiger Edelmetallkomplexe ist aus dem Stand der Technik bekannt. Siehe hierzu R. Franke, D. Selent, A. Börner, "Applied Hydroformylation", Chem. Rev., 2012, DOI:10.1 021/cr3001803; S. 5688 Schema 12 "General Method forthe Preparation of a P-Modified Rh precatalyst" und darin zitierte Literaturstellen sowie P. W. N. M. van Leeuwen, in Rhodium Catalyzed Hydroformylation, P. W. N. M. van Leeuwen, C. Claver (Hrsg.), Kluwer, Dordrecht, 2000 unter anderem S. 48 ff, S.233 ff. und darin zitierte Literaturstellen sowie K.D. Wiese und D. Obst in Top. Organomet. Chem. 2006, 18, 1-13; Springer Verlag Berlin Heidelberg 2006 S. 6 ff sowie darin zitierte Literaturstellen.

Des Weiteren betrifft die Erfindung die Verwendung der Verbindung zur Katalyse einer Hydroformylierungsreaktion. Dabei wird die erfindungsgemäße Verbindung bevorzugt als Ligand in einem erfindungsgemäßen Ligand-Metall-Komplex eingesetzt. Besonders bevorzugt ist hierbei die Hydroformylierung endständiger Olefine zu Aldehyden.

Die Erfindung betrifft ebenfalls ein Verfahren, bei dem die erfindungsgemäße Verbindung als Ligand in einem Ligand-Metall-Komplex zur Umsetzung eines Olefins zu einem Aldehyd eingesetzt wird.

Das erfindungsgemäße Verfahren umfasst die Verfahrensschritte:
a) Vorlegen eines Olefins,
b) Zugabe eines erfindungsgemäßen Komplexes oder einer erfindungsgemäßen Verbindung und einer Substanz, welche ein Metallatom ausgewählt aus Rh, Ru, Co und Ir aufweist,
c) Zuführen von Wasserstoff und Kohlenstoffmonoxid,
d) Erwärmen des Reaktionsgemisches, wobei das Olefin zu einem Aldehyd umgesetzt wird.

Hierbei können die Verfahrensschritte a) bis c) in beliebiger Reihenfolge erfolgen.

In einer bevorzugten Variante des Verfahrens ist das Metallatom Rh.

Es kann hierbei auch ein Überschuss an Liganden verwendet werden und nicht zwangsläufig jeder Ligand liegt gebunden in Form eines Ligand-Metall-Komplexes vor, sondern ist als freier Ligand im Reaktionsgemisch enthalten. Vorzugsweise liegt das molare Verhältnis der erfindungsgemäßen Verbindung zum Metallatom im Bereich von 10:1 zu 1:1, bevorzugt 8:1 zu 1:1, besonders bevorzugt 6:1 zu 2:1.

Die Reaktion wird bevorzugt bei einer Temperatur von 80 °C bis 200 °C und einem Druck von 1 bar bis 300 bar durchgeführt. Besonders bevorzugt sind eine Temperatur von 100 °C bis 160 °C und ein Druck von 5 bar bis 60 bar. Besonders bevorzugt sind eine Temperatur von 110 °C bis 130 °C und ein Druck von 10 bar bis 30 bar.

Die Edukte für die Hydroformylierung gemäß dem Verfahren der Erfindung sind Olefine oder Gemische von Olefinen, insbesondere Monoolefine mit 2 bis 24, bevorzugt 3 bis 16, besonders bevorzugt 3 bis 12 Kohlenstoffatomen mit end- oder innenständigen C-C-Doppelbindungen, wie z.B. 1-Propen, 1- oder 2-Penten, 2-Methyl-1-buten, 2-Methyl-2-buten, 3-Methyl-1-buten, 1-, 2- oder 3-Hexen, das bei der Dimerisierung von Propen anfallende C₆-Olefingemisch (Dipropen), Heptene, 2- oder 3-Methyl-1-hexene, Octene, 2-Methylheptene, 3-Methylheptene, 5-Methyl-2-hepten, 6-Methyl-2-hepten, 2-Ethyl-1-hexen, das bei der Dimerisierung von Butenen anfallende C₈-Olefingemisch (Dibuten), Nonene, 2- oder 3-Methyloctene, das bei der Trimerisierung von Propen anfallende C₉-Olefingemisch (Tripropen), Decene, 2-Ethyl-1-octen, Dodecene, das bei der Tetramerisierung oder der Trimerisierung von Butenen anfallende C₁₂-Olefingemisch (Tetrapropen oder Tributen), Tetradecene, Hexadecene, das bei der Tetramerisierung von Butenen anfallende C₁₆-Olefingemisch (Tetrabutan) sowie durch Cooligomerisierung von Olefinen mit unterschiedlicher Anzahl von Kohlenstoffatomen (bevorzugt 2 bis 4) hergestellte Olefingemische. Bevorzugt handelt es sich um Monoolefine mit einer endständigen C-C-Doppelbindung.

### Beispiele

Die Erfindung wird anhand der folgenden Ausführungsbeispiele erläutert.

### Allgemeine Arbeitsvorschriften

Alle präparativen Arbeiten erfolgten unter Anwendung der Schlenk-Technik mit Argon als Schutzgas. Toluol und Tetrahydrofuran wurden mit einem Pure Solv MD-7 System gereinigt und bis zur Verwendung unter Argon aufbewahrt. Triethylamin wurde vor dem Einsatz unter Argon von Natriumketyl destilliert. Phosphortrichlorid (Aldrich) wurde vor dem Einsatz unter Argon destilliert. Alle präparativen Arbeiten erfolgten in ausgeheizten Gefäßen. Die Aufnahme von Kernresonanzspektren erfolgte an Bruker Avance 300 bzw. Bruker Avance 400, die gaschromatografische Analyse an Agilent GC 7890A, die Elementaranalyse an Leco TruSpec CHNS und Varian ICP-OES 715, und die ESI-TOF Massenspektrometrie an Thermo Electron Finnigan MAT 95-XP und Agilent 6890 N/5973 Geräten, die halbautomatische Säulenchromatografie an einem Teledyne Isco Combiflash Rf+.

### Ligand 1

### rac-4-(Anthracen-9-yloxy)-8,9,10,11,12,13,14,15-octahydrodinaphtho[2,1-d:1',2'-f][f1,3,21dioxaphosphepin

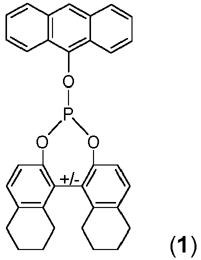

Eine bei 0 °C gerührte Mischung aus Anthron (0,385 g; 1,98 mmol), Triethylamin (2,5 ml) und THF (7 ml) wird tropfenweise mit einer Lösung von 4-Chlor-8,9,10,11,12,13,14,15-octahydrodinaphtho[2,1-*d*:1',2'-*f*][1,3,2]dioxaphosphepin (0,711 g; 1,98 mmol) in Toluol (4 ml) versetzt. Man rührt zunächst 30 min bei 0 °C und dann über Nacht bei Raumtemperatur, filtriert, entfernt das Lösungsmittel im Vakuum und trocknet den erhaltenen gelben Feststoff 2 h bei 50°C/0,1 mbar. Umkristallisation aus Heptan (12 ml, 80 °C) ergibt 0,819 g (1,58 mmol; 80%) an kristallinem Feststoff, der nach 3 h Trocknen bei 0,1 mbar/50°C noch 5 mol-% Heptan enthält.

Elementaranalyse (ber. für C₃₄H₂₉O₃P= 516,574 g/mol): C 79,22 (79,05); H 5,55 (5,66); P 5,90 (6,00)%. ESI-TOF/HRMS: *m*/e 517,19293 (*M*+H)⁺.
³¹P-NMR (CD₂Cl₂): 142,0 (s) ppm.
¹H-NMR (CD₂Cl₂): 1,67 (m, 2H); 1,88 (m, 6H); 2,41 (m, 2H); 2,76-2,96 (m, 6H); 7,10-7,28 (m, 3H); 7,38 (s, 1H); 7,54-7,65 (m, 4H); 8,08 (d, *J*_{HH}= 8,6 Hz, 2H); 8,37 (s, 1H); 8,50 (d, *J*_{HH}= 8,6 Hz, 2H) ppm.
¹³C-NMR (CD₂Cl₂): 22,9 (d); 23,0; 23,1; 28,2; 28,3; 29,6; 119,2; 119,3; 123,0; 123,4; 125,0; 126,1; 126,3; 128,4; 128,6; 129,7; 129,8; 130,0; 132,5; 135,1; 135,9; 138,5; 139,1; 143,6 (d, *J*_{CP}= 6,3 Hz); 146,1 (d, *J*_{CP}= 1,9 Hz); 146,3 (d, *J*_{CP}= 4,6 Hz) ppm.

### Ligand 2

### rac-4-([1,1':3',1"-Terphenyl]-2'-yloxy)-8,9,10,11,12,13,14,15-octahydrodinaphtho[2,1-d:1',2'-f][1,3,2]dioxaphosphepin

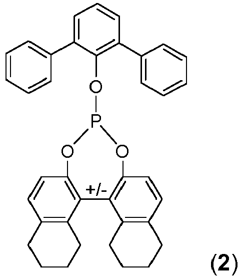

Zu einer bei 0 °C gerührten Mischung aus *rac*-5,5',6,6',7,7',8,8'-Octahydro-[1,1'-binaphthalin]-2,2'-diol (0,468 g; 1,59 mmol), Toluol (6 ml) und Triethylamin (2,03 ml) wird eine Lösung von ([1,1':3',1"-Terphenyl]-2'-yloxy)dichlorphosphan (0,552 g; 1,59 mmol) in Toluol (4 ml) getropft. Man lässt auf Raumtemperatur kommen, rührt über Nacht, filtriert und engt das Filtrat im Vakuum bis zur Trockne ein. Der erhaltene Rückstand wird 2 h bei 50°C/ 0,1 mbar getrocknet und mittels Umkristallisation aus heißem Heptan gereinigt. Ausbeute: 0,728 g (1,28 mmol; 81%).

Elementaranalyse (ber. für C₃₈H₃₃O₃P= 622,649 g/mol): C 80,41 (80,26); H 5,96 (5,85); P 5,24 (5,45)%. ESI-TOF/HRMS: *m*/e 669,22432 (*M*+H)⁺.
³¹P-NMR (CD₂Cl₂): 139,6 (s) ppm.
¹H-NMR (CD₂Cl₂): 1,42-1,66 (m, 2H); 1,69-1,96 (m, 6H); 2,64 (m, 2H); 2,55-2,89 (m, 6H); 5,39 (d, *J*_{HH}= 8,1 Hz; 1H), 6,32 (d, *J*_{HH}= 8,2 Hz; 1H); 6,83 (d, *J*_{HH}= 8,2 Hz; 1H); 6,98 (d, *J*_{HH}= 8,1 Hz; 1H); 7,31-7,36 (m, 1H); 7,39 (s, 1H); 7,42 (m, 1H); 7,51-7,61 (m, 10H) ppm.
¹³C-NMR (CD₂Cl₂): 22,7; 22,9; 23,1; 28,0; 28,1; 29,4; 29,5; 119,2; 124,9; 127,6; 127,8; 128,8; 129,5; 131,0; 131,1; 134,2; 135,4; 136,5; 136,6; 137,3; 138,7; 138,9; 145,9; 146,2 (d, *J*_{CP}= 4,2 Hz); 146,6 (d, *J*_{CP}= 9,0 Hz) ppm.

### Ligand 3 (Vergleichsbeispiel)

### 6-([1,1':3',1"-Terphenyl]-2'-yloxy)-4,8-di-tert-butyl-2,10-dimethoxydibenzo[d,f][1,3,2]dioxaphosphepin.

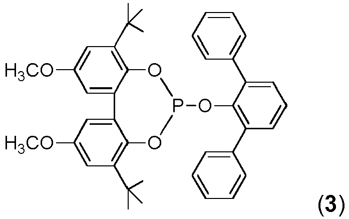

Eine Lösung von 2,6-Diphenylphenol (0,411 g; 1,65 mmol) in Toluol (8 ml) wurde mit Triethylamin (1,529 g; 15,11 mmol) versetzt und die erhaltene Mischung tropfenweise bei 0 °C zu einer Lösung von 4,8-Di-*tert*-butyl-6-chloro-2,10-dimethoxydibenzo [d,*f*],3,2]dioxaphosphepin (0,697 g; 1,65 mmol) in Toluol (6 ml) gegeben. Die Reaktionsmischung wurde über Nacht bei Raumtemperatur und für 5 h bei 70 °C gerührt. Dann wurde die Mischung filtriert und das Filtrat im Vakuum bis zur Trockne eingeengt. Der erhaltene Rückstand wurde aus Hexan (4 ml) umkristallisiert. Ausbeute: 0,417 g (0,659 mmol; 40 %).

Elementaranalyse (ber. für C₄₀H₄₁O₅P = 632,70 g/ mol) C 75,95 (75,93); H 6,52 (6,53); P 5,01 (5,00)%.
³¹P_NMR (CD₂Cl₂): 140,9 ppm.
¹H-NMR (CD₂Cl₂): 1,37 (s, 18 H); 3,84 (s, 6 H); 6,51 (d, ⁵*J*_{HH}= 3,1 Hz, 2 H, Hₐᵣₒₘ); 6,89-7,95 (m, br, 15 H, Hₐᵣₒₘ)ppm.
¹³C-NMR (CD₂Cl₂): 31,1; 35,5; 55,9; 113,0; 114,5; 125,2; 126,5-131,0; 133,8 (d, *J*_{CP}= 4,0 Hz); 141,5 (d, *J*_{CP}= 6,3 Hz); 142,6; 144,8; 156,1 ppm.
ESI-TOF/HRMS: m/e 633,27654 (M+H)⁺.

### Ligand 4 (Vergleichsbeispiel)

### 6-([1,1':3',1"-Terphenyl]-2'-yloxy)-4-(tert-butyl)-2-methoxybenzo[d]naphtho[1,2-f][1,3,2]dioxaphosphepin.

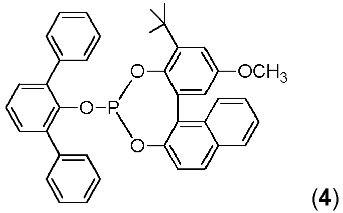

Eine Lösung von 1-(3-(*tert*-Butyl)-2-hydroxy-5-methoxyphenyl)naphthalen-2-ol (0,539 g; 1,673 mmol) in Toluol (14 ml) wurde mit Triethylamin (1,550 g; 15,320 mmol) versetzt und auf 0 °C gekühlt. Zu dieser Mischung wurde eine Lösung von 2,6-Diphenylphenoxydichlorphosphin (0,581 g; 1,673 mmol) in Toluol (3 ml) tropfenweise zugegeben. Die Reaktionsmischung wurde über Nacht gerührt und dann filtriert. Das Filtrat wurde im Vakuum bis zur Trockne eingeengt. Der erhaltene Rückstand wurde in Dichlormethan (3 ml) aufgenommen, die erhaltene Suspension über Kieselgel filtriert und das Filtrat im Vakuum zur Trockne eingeengt. Ausbeute: 0,836 g (1,401 mmol; 86 %). Elementaranalyse (ber. für C₃₉H₃₃O₄P = 596,66 g/ mol) C 78,58 (78,51); H 5,52 (5,57); P 5,28 (5,19)%.
³¹P-NMR (CD₂Cl₂): 148,9 ppm.
¹H-NMR (CD₂Cl₂): 16 (s, 9 H); 3,75 (s, 3 H); 6,81 (d, *J*_{HH} = 3,2 Hz, 1 H, Hₐᵣₒₘ); 6,95 (d, *J*_{HH} = 3,2 Hz, 1 H, Hₐᵣₒₘ); 6,98 (d, *J*_{HH} = 8,8 Hz, 1 H, Hₐᵣₒₘ); 7,14-7,19 (m, 6 H, Hₐᵣₒₘ); 7,34-7,42 (m, 3 H, Hₐᵣₒₘ); 7,44-7,49 (m, 4 H, Hₐᵣₒₘ); 7,51-7,56 (m, 2 H); 7,82 (d, *J*_{HH} = 8,8 Hz, 1 H, Hₐᵣₒₘ); 7,92 (m, 1 H, Hₐᵣₒₘ); 7,98 (m, 1 H, Hₐᵣₒₘ) ppm.
¹³C-NMR (CD₂Cl₂): 31,2 (d, *J*_{CP}= 5,7 Hz); 35,3; 55,9; 114,6 (d, *J*_{CP}= 12,3 Hz); 121,7; 125,3; 125,3; 125,8; 126,1; 126,7; 127,5; 128,4; 128,5; 128,7; 129,3; 130,3; 131,0; 132,1; 132,8; 136,5 (d, *J*_{CP}= 3,2 Hz); 138,8; 141,9 (d, *J*_{CP}= 3,8 Hz); 143,9 (d, *J*_{CP}= 3,0 Hz); 145,9 (d, *J*_{CP}= 3,0 Hz); 147,0 (d, *J*_{CP}= 6,0 Hz); 155,4 ppm.
ESI-TOF/HRMS: m/e 597,21901 (M+H)⁺.

### Ligand 5 (Vergleichsbeispiel)

### 6-(Anthracen-9-yloxy)-2,4,8,10-tetra-tert-butyldibenzo[d,f][1,3,2]dioxaphosphepin.

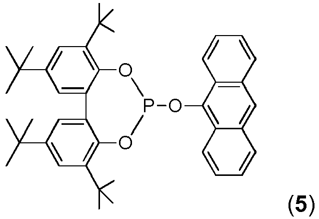

Eine gerührte Suspension von Anthracen-9-ol (0,281 g; 1,45 mmol) in Toluol (7 ml) wurde mit Triethylamin (1,344 g; 13,28 mmol) versetzt und die erhaltene Mischung tropfenweise bei 0 °C zu einer Lösung von 2,4,8,10-Tetra-*tert*-butyl-6-chlorodibenzo [*d*,*f*][1,3,2]dioxaphosphepin (0,724 g; 1,52 mmol) in Toluol (6 ml) gegeben. Die Reaktionsmischung wurde über Nacht gerührt und filtriert. Das Filtrat wurde im Vakuum bis zur Trockne eingeengt und der erhaltene Rückstand aus Hexan (4 ml) umkristallisiert. Ausbeute: 0,559 g (0,883 mmol; 61 %).

Elementaranalyse (ber. für C₄₂H₄₉O₃P = 632,78 g/ mol) C 79,83 (79,71); H 7,61 (7,81); P 5,01 (4,89) %. ³¹P-NMR (CD₂Cl₂): 140,7 ppm.
¹H-NMR (CD₂Cl₂): 1,41 (s, 18 H, C(C*H*₃)₃); 1,50 (s, 18 H, C(C*H*₃)₃); 7,40 (d, ⁵*J*_{HH}= 2,4 Hz, 2 H, Hₐᵣₒₘ); 7,43-7,56 (m, 4 H, Hₐᵣₒₘ); 7,60 (d, ⁵*J*_{HH}= 2,4 Hz, 2 H, Hₐᵣₒₘ); 8,05 (m, 2 H, Hₐᵣₒₘ); 8,34 (s, 1 H, Hₐᵣₒₘ); 8,38 (m, 2 H, Hₐᵣₒₘ) ppm.
¹³C-NMR (CD₂Cl₂): 31,2; 31,2; 31,8; 35,1; 35,7; 123,1; 123,7 (d, *J*_{CP}= 5,3 Hz); 124,8; 125,0; 125,8; 126,0; 127,2; 128,4; 132,6; 133,3 (d, *J*_{CP}= 3,7 Hz); 141,0; 143,5; 145,8 (d, *J*_{CP}= 6,2 Hz); 147,7 ppm.
ESI-TOF/HRMS: m/e 633,34934 (M+H)⁺.

### Durchführung der Katalyseversuche

Die Hydroformylierung wurde in mit Druckkonstanthaltung, Gasflussmessung, Begasungsrührer und Druckpipette ausgestattete 200 ml-Autoklaven der Fa. Premex Reactor AG, Lengau, Schweiz, durchgeführt. Das als Lösungsmittel verwendete Toluol wurde mit einem Pure Solv MD-7 System gereinigt und unter Argon aufbewahrt. Das als Substrat eingesetzte Substrat n-Octene (Octenisomerengemisch aus 1-Octen: ∼3 %; *cis*+*trans*-2-Octen: 48 %; *cis*+*trans-*3*-*Octen*:* 29%; *cis*+*trans-*Octen*-*4*:* 17 %; gerüstisomere Octene: 3 %) wurde mehrere Stunden über Natrium am Rückfluß erhitzt und unter Argon destilliert.

Für die Versuche wurden im Autoklaven unter Argonatmosphäre Lösungen der Katalysatorvorstufe und des Liganden gemischt. Als Katalysatorvorstufe kam [(acac)Rh(COD)] (Umicore, acac = Acetylacetonat-Anion; COD = 1,5-Cyclooctadien) zum Einsatz. Für Versuche mit einer Konzentration von 100 ppm-m Rhodium wurden 10 ml einer 4,31 millimolaren Lösung in den Autoklaven gegeben. Anschließend wurde die einem Verhältnis L/Rh = 5:1 entsprechende Masse des Liganden in 10 ml Toluol gelöst und zugemischt. Durch Zugabe von weiterem Toluol wurde das Anfangsvolumen der Katalysatorlösung auf 41,0 ml eingestellt. In eine druckfeste Pipette wurde eingefüllt: n-Octene (10,70 g; 95,35 mmol). Der Autoklav wurde mit Synthesegas (Linde; H₂ (Qualität 5.0): CO (Qualität 4.7) = 1:1) auf einen Gesamtdruck von 42 bar gebracht und auf 120°C aufgeheizt. Nach Erreichen der Reaktionstemperatur wurde der Synthesegasdruck im Autoklaven auf 48,5 bar erhöht und das Olefingemisch mit einem in der Druckpipette eingestellten Druck von ca. 51,5 bar in den Autoklaven gepresst. Die Reaktion wurde bei konstantem Druck von 20 bar (Nachdruckregler der Fa. Bronkhorst, NL) über 4 h geführt. Der Autoklav wurde nach Ablauf der Reaktionszeit auf Zimmertemperatur abgekühlt, unter Rühren entspannt und mit Argon gespült. Jeweils 1 ml der Reaktionsmischungen wurden unmittelbar nach Abschalten des Rührers entnommen, mit 5 ml Pentan verdünnt und gaschromatographisch analysiert: HP 5890 Series II plus, PONA, 50 m x 0,2 mm x 0,5 µm.

Die Ergebnisse sind in der nachfolgenden Tabelle gezeigt.

| **Ligand** | **Ausbeute [%]** | **Selektivität [%]** |
|---|---|---|
| 1* | 99 | 32,0 |
| 2* | 99 | 26,5 |
| 3 | 29 | 32,1 |
| 4 | 69 | 33,9 |
| 5 | 86 | 31,6 |

| | | |
|---|---|---|
| * erfindungsgemäße Verbindung Selektivität % = n / (n + i) | | |

Die Vergleichsverbindungen 3 bis 5 erzielen zwar ähnlich gute n-Selektivitäten wie die erfindungsgemäßen Verbindungen, allerdings verbunden mit einer deutlich schlechteren Ausbeute.

## Patentansprüche

1. Verbindung ausgewählt aus einer der Strukturen gemäß den Formeln (1) und (2),

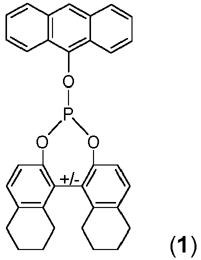

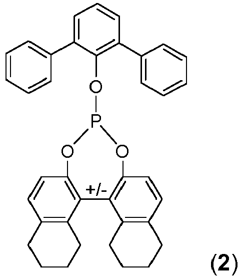

2. Komplex umfassend eine Verbindung nach Anspruch 1 und ein Metallatom ausgewählt aus Rh, Ru, Co und Ir.

3. Verwendung einer Verbindung nach Anspruch 1 zur Katalyse einer Hydroformylierungsreaktion.

4. Verwendung eines Komplexes nach Anspruch 2 zur Katalyse einer Hydroformylierungsreaktion.

5. Verfahren zur Herstellung eines Aldehyds, umfassend die Verfahrensschritte:
a) Vorlegen eines Olefins,
b) Zugabe eines Komplexes nach Anspruch 2 oder einer Verbindung nach Anspruch 1 und einer Substanz, welche ein Metallatom ausgewählt aus Rh, Ru, Co und Ir aufweist,
c) Zuführen von Wasserstoff und Kohlenstoffmonoxid,
d) Erwärmen des Reaktionsgemisches, wobei das Olefin zu einem Aldehyd umgesetzt wird.

6. Verfahren nach Anspruch 5,
wobei das molare Verhältnis der Verbindung nach Anspruch 1 zu dem Metallatom nach Anspruch 5 im Bereich von 10:1 bis 1:1 liegt.

## Claims

1. Compound selected from one of the structures according to formulae **(1)** and **(2):**

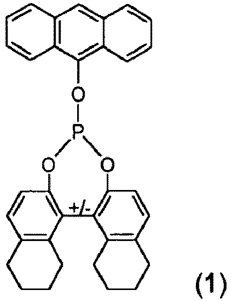

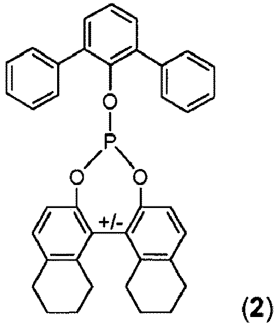

2. Complex comprising a compound according to Claim 1 and a metal atom selected from Rh, Ru, Co and Ir.

3. Use of a compound according to Claim 1 for catalysis of a hydroformylation reaction.

4. Use of a complex according to Claim 2 for catalysis of a hydroformylation reaction.

5. Method for preparing an aldehyde comprising the method steps of:
a) initially charging an olefin,
b) adding a complex according to Claim 2 or a compound according to Claim 1 and a substance having a metal atom selected from Rh, Ru, Co and Ir,
c) feeding in of hydrogen and carbon monoxide,
d) heating the reaction mixture whereupon the olefin is converted to an aldehyde.

6. Method according to Claim 5,
wherein the molar ratio of the compound according to Claim 1 to the metal atom according to Claim 5 is in the range from 10:1 to 1:1.

## Revendications

1. Composé choisi parmi une des structures selon les formules **(1)** et **(2)** :

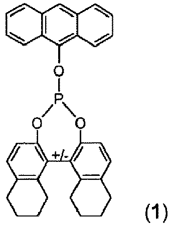

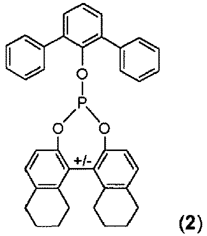

2. Complexe comprenant un composé selon la revendication 1 et un atome métallique choisi parmi Rh, Ru, Co et Ir.

3. Utilisation d'un composé selon la revendication 1 pour la catalyse d'une réaction d'hydroformylation.

4. Utilisation d'un complexe selon la revendication 2 pour la catalyse d'une réaction d'hydroformylation.

5. Procédé pour la préparation d'un aldéhyde, comprenant les étapes de Procédé :
a) disposition au préalable d'une oléfine,
b) addition d'un complexe selon la revendication 2 ou d'un composé selon la revendication 1 et d'une substance qui comporte un atome métallique choisi parmi Rh, Ru, Co et Ir,
c) apport d'hydrogène et de monoxyde de carbone,
d) chauffage du mélange réactionnel, de sorte que l'oléfine est convertie en un aldéhyde.

6. Procédé selon la revendication 5,
dans lequel le rapport molaire du composé selon la revendication 1 à l'atome métallique selon la revendication 5 se situe dans la plage de 10:1 à 1:1.
